# EUROPÄISCHE PATENTSCHRIFT

(11) **EP 0 521 501 B1**
(45) Veröffentlichungstag und Bekanntmachung des Hinweises auf die Patenterteilung: **13.01.1999**
(21) Anmeldenummer: 92111209.0
(22) Anmeldetag: 02.07.1992
(51) Int. Cl.: A61B 17/39

(54) **Elektrochirurgisches Behandlungsinstrument**
Electrosurgical treatment instrument
Instrument de traitement electrochirurgical

(30) Priorität: 04.07.1991 DE 4122219
(43) Veröffentlichungstag der Anmeldung: 07.01.1993
(73) Patentinhaber: DELMA ELEKTRO-UND MEDIZINISCHE APPARATEBAU GESELLSCHAFT mbH, D-78505 Tuttlingen (DE)
(72) Erfinder: Hagen, Alfred, W-7200 Tuttlingen 16 (DE)
(74) Vertreter: Morgan, James Garnet

(56) Entgegenhaltungen:
- EP-A- 0 067 680
- EP-A- 0 397 910
- DE-A- 3 510 586
- DE-C- 3 423 356
- FR-A- 2 502 935
- GB-A- 2 165 761
- US-A- 4 014 343
- US-A- 4 682 596

## Beschreibung

Die Erfindung betrifft ein elektrochirurgisches Behandlungsinstrument nach dem Oberbegriff des Patentanspruchs 1.

Bei einem derartigen bekannten Behandlungsinstrument (DE 34 23 356 C2) sind die beiden Elektroden in bezug auf die Achse des Behandlungsinstrumentes nebeneinander angeordnet und als Branchen einer Pinzette ausgebildet. Damit lassen sich Gewerbebereiche eines Patienten, die mit den Elektroden pinzettenartig erfaßt werden können, einwandfrei bipolar koagulieren.

Sollen jedoch Gewebebereiche koaguliert werden, die sich nicht auf einfache Weise pinzettenartig erfassen lassen, so erfordert eine einwandfreie Koagulation sehr viel Aufmerksamkeit und Geschick des Operateurs, da er den für eine bipolare Koagulation erforderlichen Elektrodenabstand durch Zusammendrücken der pinzettenartigen Elektroden einstellen und gegebenenfalls aufgrund des Koagulationsergebnisses anpassen muß.

Die Aufgabe der vorliegenden Erfindung besteht darin, ein weiteres elektrochirurgisches Behandlungsinstrument der eingangs genannten Art zu schaffen; insbesondere soll es ermöglicht werden, einwandfreie bipolare Koagulationen durchzuführen, ohne den Elektrodenabstand während eines chirurgischen Eingriffs einstellen zu müssen und ohne daß eine Gefahr besteht, daß das Gewebe sich an den Elektroden verhakt.

Diese Aufgabe wird durch die Merkmale des kennzeichnenden Teils des Anspruchs 1 gelöst.

Auf diese Weise genügt es, wenn zur Koagulation die Seiten- oder Umfangsfläche des stift- oder zapfenförmigen Körpers so an das zu koagulierende Gewebe angelegt wird, daß beide Elektroden sich in einen Koagulationsstrom übertragendem Kontakt mit dem Gewebe befinden. Wahlweise kann die zentral innerhalb der beiden Koagulationselektroden isoliert angeordnete Schneidelektrode aktiviert werden, die vorne aus dem Koagulationskörper um ein definiertes Stück vorstehen kann und vorzugsweise als ein nadelartiger Draht ausgebildet ist.

Der zapfenförmige Körper weist dabei vorzugsweise nur abgerundete Flächen und keine Ecken und Kanten auf; insbesondere ist er kreiszylindrisch und am vorderen Ende verjüngt und/oder abgerundet.

Dadurch, daß die Mantel- bzw. Umfangsfläche des Koagulationskörpers glatt, d.h. weitgehend vorsprungsfrei und abgerundet ausgebildet ist, ist ein einwandfreies Gleiten auf der Oberfläche des Gewebes möglich und Verletzungen durch Verschieben des Körpers entlang des Gewebes werden ebenso vermieden wie Verhakungen des Koagulationskörpers am Gewebe.

Vorteilhaft an dem erfindungsgemäßen Behandlungsinstrument ist weiter, daß der für eine bipolare Koagulation erforderliche Abstand der Koagulationselektroden voneinander genau festgelegt ist. Der Operateur kann sich also während der Behandlung vollständig auf den erforderlichen Eingriff konzentrieren, insbesondere braucht er mit den fest beabstandeten Elektroden nur an dem zu koagulierenden Gewebe entlangzufahren, ohne auf die Einhaltung eines geeigneten Elektrodenabstandes achten zu müssen.

Außerdem ermöglicht die seitliche Lage des Koagulationsbereiches eine bequeme Handhabung und Führung des Behandlungsinstrumentes.

Vorteilhafte Weiterbildungen entnimmt man den Ansprüchen 2 bis 12.

Vorteilhafterweise soll der Grad des Vorstehens der Schneidelektrode vor dem Koagulationskörper gemäß Anspruch 13 veränderbar sein. Diese Ausführungsform kann nach den Ansprüchen 14 bis 17 weitergebildet werden.

Nachdem eine Schneidelektrode sich mit der Zeit abnutzt oder auch beim Gebrauch beschädigt werden kann, soll sie gemäß Anspruch 18 vorzugsweise abnehmbar, d.h. auch auswechselbar sein.

Vorteilhafte Weiterbildungen dieser Ausführungsform sind durch die Ansprüche 19 bis 22 gekennzeichnet.

Die vorteilhafte Kombination von zwei Koagulationselektroden und einer fest angeordneten Schneidelektrode ist durch Anspruch 23 definiert.

Eine bevorzugte Steuerschaltung für das erfindungsgemäße Behandlungsinstrument läßt sich den Ansprüchen 24 und 25 entnehmen. Durch die Überlagerung des Hochfrequenzstroms und eines niederfrequenten Steuerstroms können die gleichen leitungen, die für die Zuführung des Hochfrequenzstroms verwendet werden, auch für die Führung des Steuerstroms dienen.

Die vordere der beiden Koagulationselektroden ist vorzugsweise die auch beim Schneiden wirksame Bezugselektrode.

Die Erfindung wird im folgenden beispielsweise anhand der Zeichnung näher beschrieben; in dieser zeigt:
- Fig. 1: eine Draufsicht auf ein elektrochirurgisches Hochfrequenz-Behandlungsinstrument mit Anschlußkabel und Stecker, jedoch ohne die Hochfrequenz-Schaltelektronik,
- Fig. 2: einen schematischen Längsteilschnitt durch den vorderen Teil des Behandlungsinstrumentes nach Fig. 1, welcher die verschiedenen Elektroden enthält, und
- Fig. 3: ein schematisches, stark vereinfachtes Schaltbild der Hochfrequenz-Schaltelektronik sowie der Hochfrequenz- und Steuerstromzuleitungen zu den Elektroden des Behandlunesinstrumentes nach den Fig. 1 und 2.

In den verschiedenen Figuren der Zeichnung sind einander entsprechende Bauteile mit gleichen Bezugszahlen bezeichnet.

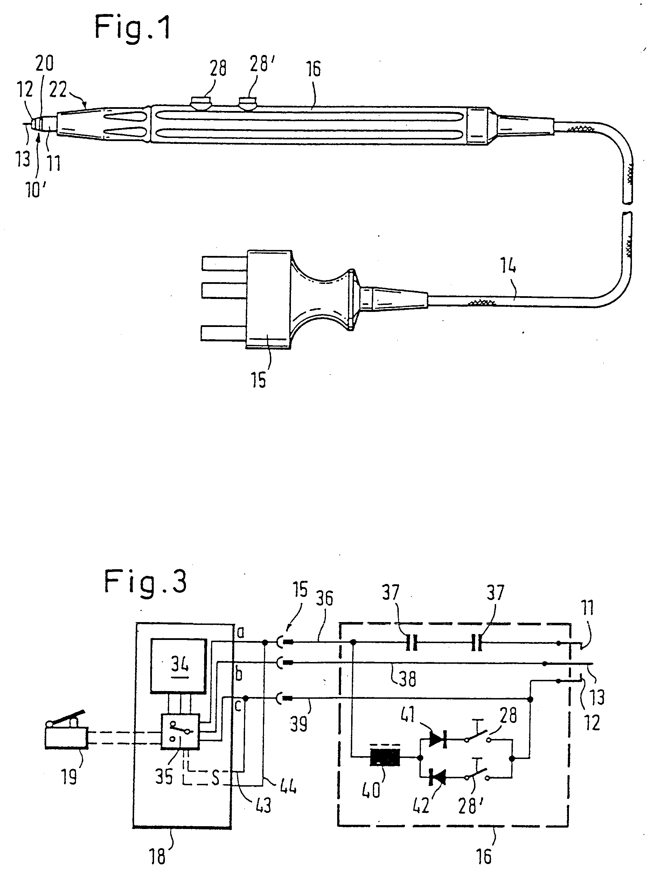

Das in Fig. 1 dargestellte elektrochirurgische Behandlungsinstrument weist einen Halter 16 auf, an dessen in Fig. 1 linkem, proximalen Ende eine Kappe 22 mittels eines Gewindes, Bajonettverschlusses oder einer Verschiebevorrichtung angeordnet ist, aus der nach vorn eine Elektrodenanordnung mit einer hinteren hülsenförmigen Koagulationselektrode 11, einer vorderen ringförmigen Bezugselektrode 12 und einer von dieser nach vorn vorspringenden Schneidelektrode 13 vorsteht. Die Baueinheit aus der Koagulationselektrode 11, der Bezugselektrode 12 und einem dazwischen angeordneten Isolierring 20 bildet einen im wesentlichen kreiszylinderförmigen zapfenartigen Koagulationskörper 10'.

An das distale Ende des Halters 16 ist ein Kabel 14 angeschlossen, das an seinem anderen Ende mit einem Stecker 15 zum Anschluß an eine Hochfrequenz-Schaltelektronik 18 (Fig. 4) versehen ist; die Lösbarkeit des Kabels 14 von der Hochfrequenz-Schaltelektronik 18 ist erforderlich, da solche Instrumente zum Sterilisieren von der Elektronik entfernt werden müssen. Aus dem gleichen Grunde ist das Kabel 14 auch mit dem distalen Ende des Halters 16 durch eine Steckverbindung verbunden.

Zum wahlweisen Einschalten des Behandlungsinstrumentes für Schneid- oder Koagulationsbetrieb sind am Halter 16 hintereinander zwei Tastschalter 28, 28' vorgesehen. Es könnte aber auch ein Schiebeschalter verwendet werden. Weiter könnte die Betätigung über einen Fußschalter 19 (Fig. 3) erfolgen, der direkt an die Hochfrequenz-Schaltelektronik 18 angeschlossen ist.

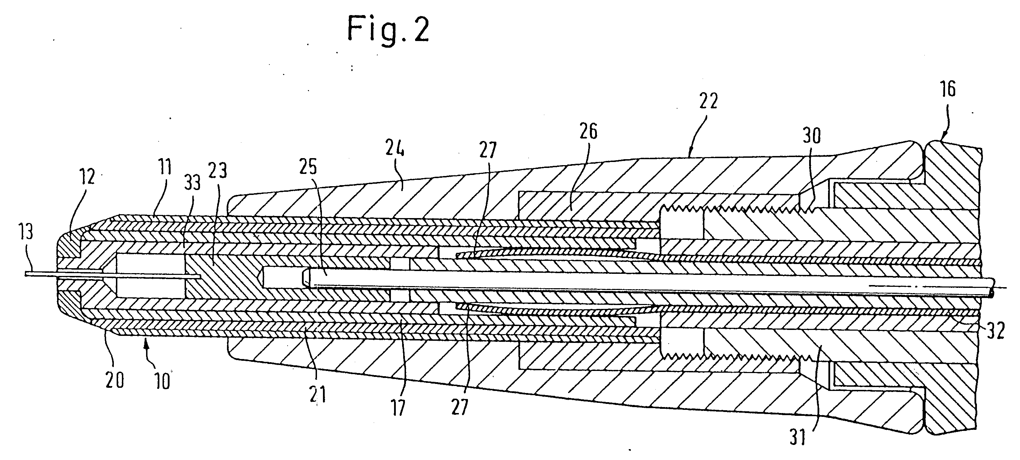

Nach Fig. 2 weist die als leicht konischer Hohlkörper ausgebildete Kappe 22 eine isolierende Griffhülse 24 auf, in die eine aus Metall bestehende Innengewindehülse 26 fest eingesetzt ist, in die koaxial die als kreiszylinderförmige Hülse ausgebildete Koagulationselektrode 11 eingesetzt ist, welche ihrerseits mit ihrem proximalen Ende aus der Griffhülse 24 vorsteht. In die Koagulationselektrode 11 ist ein Isolierrohr 21 mit einem vorderen Isolierring 20 koaxial eingesetzt, der an der vorderen Stirnfläche der Koagulationselektrode 11 und an der hinteren Stirnfläche einer vor der Koagulationselektrode 11 angeordneten Bezugselektrode 12 anliegt, die als Metallring mit nach vorn verjüngter und abgerundeter Umfangsfläche ausgebildet ist. Die Umfangsflächen der Bezugselektrode 12, des Isolierringes 20 und der Koagulationselektrode 11 gehen stetig und stufenlos ineinander über.

Die Bezugselektrode 12 ist an ihrer hinteren Seite mit einem Metallrohr 17 verbunden, das koaxial in das Isolierrohr 21 eingesetzt ist und an seinem hinteren Ende eine Buchse für die Aufnahme eines am Halter 16 befestigten federnden Steckerkontaktes 27 bildet.

In dem Metallrohr 17 ist ein weiteres Isolierrohr 33 angeordnet, das die Schneidelektrode 13 und deren an ihrem hinteren Ende angeordnete Anschlußbuchse 23 aufnimmt.

Vom vorderen Ende des aus Isoliermaterial bestehenden Halters 16 steht ein mit einem Außengewinde 30 versehenes Metallrohr 31 vor, auf das die Kappe 22 aufgeschraubt ist. Innerhalb des auch als isolierte elektrische Zuleitung zur Koagulationselektrode 11 dienenden Metallrohres 31 sind außerdem die isoliert geführten Zuleitungen 25 und 32 für die Schneidelektrode 13 bzw. die Bezugselektrode 12 angeordnet.

Die Zuleitung 25 für die Schneidelektrode 13 ist an ihrem vorderen Ende als Stecker ausgebildet, der in die Buchse 23 der Schneidelektrode 13 lösbar eingesteckt ist.

Um das Behandlungsinstrument gebrauchsfertig zusammenzubauen, wird zunächst die Schneidelektrode 13 mit der Buchse 23 auf den Stecker am vorderen Ende der Zuleitung 25 aufgesteckt. Anschließend wird die Schraubkappe 22 so auf das vordere Ende des Halters 16 aufgeschoben, daß die Schneidelektrode 13 in das Isolierrohr 33 und der Steckerkontakt 27 in das Metallrohr 17 eintritt sowie die Innengewindehülse 26 der Schraubkappe 22 mit der Außengewindehülse 31 des Halters 16 in Gewindeeingriff gelangt und damit auf den Halter 16 aufgeschraubt werden kann.

Beim Aufschrauben tritt die Schneidelektrode 13 durch das im Inneren des Metallrohrs 17 angeordnete, sich in der zentralen Öffnung der Elektrode 12 verjüngende Isolierrohr 33 hindurch mehr und mehr aus der zentralen Öffnung der Bezugselektrode 12 nach vorn hervor. Durch unterschiedlich weites Aufschrauben der Schraubkappe 22 kann somit erreicht werden, daß die Schneidelektrode 13 mehr oder weniger weit aus dem vorderen Ende des Koagulationskörpers 10 vorspringt.

Wie Fig. 3 schematisch zeigt, erfolgt die Versorgung der Elektroden 11, 12, 13 mit HF-Spannung bzw. HF-Strom von einem HF-Generator 34, der Bestandteil der Hochfrequenz-Schaltelektronik 18 ist. Der HF-Generator 34 kann sowohl einen zum Schneiden als auch einen zum Koagulieren geeigneten Hochfrequenzstrom liefern, was durch ein Umschaltgerät 35 bestimmt wird. Das Umschaltgerät 35 legt die geeignete HF-Spannung über Ausgänge a, b, c und den Stecker 15 entweder an das Koagulationselektrodenpaar 11, 12 oder Schneidelektrodenpaar 12, 13.

In der zur Koagülationselektrode 11 geführten Zuleitung 36 sind innerhalb des Halters 16 ein oder Zwei als Hochpaß dienende Kondensatoren 37 angeordnet. Die HF-Ausgänge b, c sind dagegen durch Zuleitungen 38, 39 direkt mit der Schneidelektrode 13 bzw. der Bezugselektrode 12 verbunden. Von der Zuleitung 36 zweigt vor den Kondensatoren 37 eine als Tiefpaß für einen niederfrequenten Steuerstrom dienende Drossel 40 ab, die über zwei gegensinnig parallel zueinander geschaltete, als Gleichrichter dienende Dioden 41, 42 und diesen nachgeschaltete Tastschalter 28, 28' mit der Zuleitung 39 zur vorzugsweise auf Erdpotential liegenen Bezugselektrode 12 verbunden ist. Außerdem sind zwei Leitungen 43, 44 eines Steuer-Wechselspannungsausganges S der Hochfrequenz-Schaltelektronik 18 an die HF-Ausgänge a, c angeschlossen.

Wird beim Betrieb des in der oben beschriebenen Weise zusammengesetzten Behandlungsinstrumentes vom Operateur z.B. der Tastschalter 28 für Schneidbetrieb betätigt, so wird die Spannung am Steuerausgang S kurzgeschlossen und es fließt ein niederfrequenter, kleiner Steuerstrom in einer Richtung über die Drossel 40 und die Diode 41 in das Schaltgerät 35, welches daraufhin die für einen Schneidbetrieb erforderliche HF-Spannung auf die HF-Ausgänge b, c legt, die mit der Schneideelektrode 13 bzw. der Bezugselelektrode 12 verbunden sind.

Wird andererseits für den Koagulationsbetrieb der Tastschalter 28' geschlossen, so fließt ein entsprechender niederfrequenter Steuerstrom in entgegengesetzer Richtung über die Drossel 40 und die Diode 42, woraufhin das Schaltgerät 35 eine für bipolare Koagulation geeignete HF-Spannung an die HF-Ausgänge a, c anlegt, so daß der Koagulationselektrode 11 und der jetzt ebenfalls als Koagulationselektrode dienenden Bezugselektrode 12 die für die Koagulation erforderliche HF-Spannung bzw. der für die Koagulation erforderliche HF-Strom zugeführt wird.

Während eines chirurgischen Eingriffs braucht der Operateur also nur jeweils den einen oder den anderen Tastschalter 28 zu drücken, um mit dem Behandlungsinstrument wahlweise schneiden oder koagulieren zu können.

### Liste der Bezugszeichen

- 10: Koagulationskörper
- 11: Koagulationselektrode
- 12: Bezugselektrode
- 13: Schneidelektrode
- 14: Kabel
- 15: Stecker
- 16: Halter
- 17: Metallrohr
- 18: Hochfrequenz-Schaltelektronik
- 19: Fußschalter
- 20: Isolierring
- 21: Isolierrohr
- 22: Kappe
- 23: Buchse
- 24: Griffhülse
- 25: axiale Zuleitung
- 26: Innengewindehülse
- 27: Steckerkontakt
- 28, 28': Tastschalter
- 29: Anschlußbuchse
- 30: Außengewinde
- 31: Metallrohr
- 32: Zuleitung
- 33: Isolierrohr
- 34: HF-Generator
- 35: Umschaltgerät
- 36: Zuleitung
- 37: Kondensator
- 38: Zuleitung
- 39: Zuleitung
- 40: Drossel
- 41: Diode
- 42: Diode
- 43: Leitung
- 44: Leitung

## Patentansprüche

1. Elektrochirurgisches Behandlungsinstrument mit einem an einen Hochfrequenzgenerator (34) anschließbaren Halter (16; 16'), an dessen proximalem Ende wenigstens zwei mit dem Gewebe eines Patienten in Berührung bringbare, gegeneinander isolierte, durch eine vom Hochfrequenzgenerator gelieferte HF-Spannung gespeiste Koagulationselektroden (11, 12; 11', 12') und eine Schneidelektrode (13) vorgesehen sind,
**dadurch gekennzeichnet,**
daß die beiden Elektroden (11, 12) ring- oder hülsenartig ausgebildet, ineinander angeordnet und durch eine ringförmige isolierende Trennschicht (20) voneinander getrennt sind, wobei die beiden Elektroden (11, 12) zumindest teilweise an den Seitenflächen eines stift- oder zapfenförmigen Körpers (10) ausgebildet sind und der Abstand ihrer Außenflächen so gering ist, daß beim Anlegen der Elektroden (11, 12) an das Gewebe eines Patienten ein Koagulationsstrom fließt, und daß die Schneidelektrode (13) im Innenraum der beiden hülsenartig ausgebildeten Elektroden (11, 12) angeordnet ist.

2. Behandlungsinstrument nach Anspruch 1,
**dadurch gekennzeichnet,**
daß der Körper (10) im wesentlichen zylindrisch ist.

3. Behandlungsinstrument nach Anspruch 2,
**dadurch gekennzeichnet,**
daß der Körper (10) am proximalen Ende sich nach vom verjüngend ausgebildet ist.

4. Behandlungsinstrument nach einem der vorhergehenden Ansprüche,
**dadurch gekennzeichnet,**
daß die Seitenflächen des Körpers (10) als glatte Mantelflächen ausgebildet sind.

5. Behandlungsinstrument nach einem der vorhergehenden Ansprüche,
**dadurch gekennzeichnet,**
daß die beiden Elektroden (11, 12) hintereinander angeordnet sind.

6. Behandlungsinstrument nach Anspruch 5,
**dadurch gekennzeichnet,**
daß die vordere Elektrode (12) ein kugel-, parabol- oder konusartiger Metallring und die hintere Elektrode (11) eine zylindrische Metallhülse ist.

7. Behandlungsinstrument nach Anspruch 6,
**dadurch gekennzeichnet,**
daß die vordere Elektrode (12) und die hintere Elektrode (11) konzentrisch zueinander sind.

8. Behandlungsinstrument nach einem der Ansprüche 5 bis 7,
**dadurch gekennzeichnet,**
daß zwischen den Elektroden (11, 12) ein Isolierring (20) vorgesehen ist.

9. Behandlungsinstrument nach Anspruch 8,
**dadurch gekennzeichnet,**
daß der Isolierring (20) zu den Elektroden (11, 12) konzentrisch ist.

10. Behandlungsinstrument nach einem der Ansprüche 5 bis 9,
**dadurch gekennzeichnet,**
daß sich innerhalb der als Hülse ausgebildeten hinteren Elektrode (11) ein nach hinten gerichteter, einen kleineren Durchmesser aufweisender, rohrförmiger Fortsatz (21) des Isolierrings (20) erstreckt, in welchem ein sich von der vorderen Elektrode (12) nach hinten erstreckendes Metallrohr (17) angeordnet ist.

11. Behandlungsinstrument nach Anspruch 10,
**dadurch gekennzeichnet,**
daß der nach hinten gerichtete Fortsatz (21) des Isolierrings (20) im Paßsitz innerhalb der hinteren Elektrode (11) angeordnet ist.

12. Behandlungsinstrument nach Anspruch 10 oder 11,
**dadurch gekennzeichnet,**
daß das sich von der vorderen Elektrode (12) nach hinten erstreckende Metallrohr (17) im Paßsitz im Fortsatz (21) des Isolierrings (20) angeordnet ist.

13. Elektrochirurgisches Behandlungsinstrument nach einem der vorangehenden Ansprüche,
**dadurch gekennzeichnet,**
daß die ring- oder hülsenartige Elektrode (11) relativ zu der axial fest am Halter (16) angeordneten Schneidelektrode (13) axial verstellbar ist.

14. Behandlungsinstrument nach einem der vorhergehenden Ansprüche,
**dadurch gekennzeichnet,**
daß die Elektroden (11, 12) und die Trennschichten (20, 21) mit den Befestigungselementen eine Baueinheit bilden, die auf dem proximalen Ende des Halters (16) angeordnet ist.

15. Behandlungsinstrument nach Anspruch 14,
**dadurch gekennzeichnet,**
daß die Baueinheit als Kappe (22) ausgebildet ist.

16. Behandlungsinstrument nach Anspruch 9 oder 10,
**dadurch gekennzeichnet,**
daß die Kappe (22) gegenüber dem Halter (16) axial verstellbar ist.

17. Behandlungsinstrument nach Anspruch 16,
**dadurch gekennzeichnet,**
daß die Kappe (22) gegenüber dem Halter (16) schraubbar ist.

18. Elektrochirurgisches Behandlungsinstrument nach einem der vorhergehenden Ansprüche,
**dadurch gekennzeichnet,**
daß die Schneidelektrode (13) abnehmbar angeordnet ist.

19. Behandlungsinstrument nach einem der vorhergehenden Ansprüche,
**dadurch gekennzeichnet,**
daß die vordere ring- oder hülsenartige Elekrode die Bezugselektrode (12) ist.

20. Behandlungsinstrument nach einem der vorhergehenden Ansprüche,
**dadurch gekennzeichnet,**
daß die Schneidelektrode (13) durch die hohl ausgebildeten anderen beiden Elektroden (11, 12) und die dazwischen befindliche Isolierung (20, 21) axial hindurchgeführt ist.

21. Behandlungsinstrument nach einem der Ansprüche 18 bis 20,
**dadurch gekennzeichnet,**
daß die Schneidelektrode (13) über eine Steckverbindung (23, 25) an einer fest im Halter (16) angeordneten axialen Zuleitung (25) angebracht ist.

22. Behandlungsinstrument nach einem der vorhergehenden Ansprüche,
**dadurch gekennzeichnet,**
daß in die ring- oder hülsenartige vordere Elektrode (12) ein Isolierrohr (33) eingesetzt ist, durch das die Schneidelektrode (13) axial hindurchgeht.

23. Behandlungsinstrument nach einem der vorhergehenden Ansprüche,
**dadurch gekennzeichnet,**
daß konzentrisch zueinander zwei axial versetzte hohle Koagulationselektroden (11, 12) und innerhalb dieser eine vorn vorstehende Schneidelektrode (13) angeordnet sind.

24. Behandlungsinstrument nach einem der vorhergehenden Ansprüche,
**dadurch gekennzeichnet,**
daß wenigstens eines der Hochfrequenzzuleitungspaare (36, 39) zusätzlich von einem niederfrequenten Steuerstrom beaufschlagt ist, der durch einen Hochpaß (37) von der aktiven Elektrode (11) ferngehalten und zu einer am Halter (16) vorgesehenen Steuerschalteranordnung (28, 28') geführt ist, welche durch einen Tiefpaß (40) von der Hochfrequenz getrennt ist und durch Schalten des Steuerstroms in der einen oder anderen Richtung ein Schaltsignal an ein an den hochfrequenzgenerator (34) angeschlossenes Umschaltgerät (35) liefert, welches den passenden Hochfrequenzstrom je nach Richtung des Steuerstroms an das Koagulationselektrodenpaar (11, 12) oder das Schneidelektrodenpaar (12, 13) anlegt.

25. Behandlungsinstrument nach Anspruch 24,
**dadurch gekennzeichnet,**
daß der Steuerstrom ein Wechselstrom ist und die Steuerschalteranordnung zwei jeweils mit einem entgegengesetzt geschalteten Gleichrichter (41, 42) in Reihe liegende, parallel geschaltete Schalter (28, 28') aufweist und daß im Umschaltgerät (35) eine Schaltanordnung vorhanden ist, die je nachdem, welcher der beiden Steuerschalter (28, 28') geschlossen wird, entweder das Koagulationselektrodenpaar (11, 12) oder das Schneidelektrodenpaar (12, 13) mit dem dafür vorgesehenen Hochfrequenzstrom beaufschlagt.

## Claims

1. Electrosurgical treatment instrument comprising a holder (16; 16') connectable to a radio frequency generator (34) with at least two coagulation electrodes (11, 12; 11', 12') which are provided at the proximal end of the holder, which can be brought into contact with the tissue of a patient, and which are insulated relative to one another and fed by an rf voltage delivered from the radio frequency generator, characterised in that the two electrodes (11, 12) are of ring-like or sleeve-like design, are arranged inside one another and separated from one another by a ring-like insulating separation layer (20), with the two electrodes (11, 12) formed at least partly at the side surfaces of a pin-like or spigot-like body (10); and with the spacing of their outer surfaces being so small that on contacting the electrodes with the tissue of a patient, a coagulation current flows; and in that the cutting electrode is arranged in the inner space of the two electrodes (11, 12) of sleeve-like design.

2. Treatment instrument in accordance with claim 1, characterised in that the body (10) is substantially cylindrical.

3. Treatment instrument in accordance with claim 2, characterized in that the body (10) tapers forwardly at the proximal end.

4. Treatment instrument in accordance with one of the preceding claims, characterised in that the side surfaces of the body (10) are formed as smooth envelope surfaces.

5. Treatment instrument in accordance with one of the preceding claims, characterised in that the two electrodes (11, 12) are arranged behind one another.

6. Treatment instrument in accordance with claim 5, characterised in that the front electrode (12) is a spherical, parabolic or conical metal ring and the rear electrode (11) is a cylindrical metal sleeve.

7. Treatment instrument in accordance with claim 6, characterized in that the front electrode (12) and the rear electrode (11) are concentric to one another.

8. Treatment instrument in accordance with one of the claims 5 to 7, characterised in that an insulating ring (20) is provided between the electrodes (11, 12).

9. Treatment instrument in accordance with claim 8, characterized in that the insulating ring (20) is concentric to the electrodes (11, 12).

10. Treatment instrument in accordance with one of the claims 5 to 9, characterised in that a rearwardly directed tubular projection (21) of the insulating ring (20), in which a metal tube (17) is arranged, which extends rearwardly from the front electrode (12) and has a smaller diameter, extends within the rear electrode (11) which is formed as a sleeve.

11. Treatment instrument in accordance with claim 10, characterized in that the rearwardly directed projection (21) of the insulating ring (20) is arranged in a fitted seat within the rear electrode (11).

12. Treatment instrument in accordance with claim 10 or claim 11, characterised in that the metal tube (17) extending rearwardly from the front electrode (12) is arranged in the fitted seat in the projection (21) of the insulating ring (20).

13. Electrosurgical treatment instrument in accordance with one of the preceding claims, characterized in that the ring or sleeve-like electrode (11) is axially adjustable relative to the cutting electrode (13), which is axially fixedly arranged at the holder (16).

14. Treatment instrument in accordance with one of the preceding claims, characterized in that the electrodes (11, 12) and the separation layers (20, 21) form a constructional unit with the attachment elements which is arranged at the proximal end of the holder (16).

15. Treatment instrument in accordance with claim 14, characterized in that the constructional unit is formed as a cap (22).

16. Treatment instrument in accordance with claim 9 or claim 10, characterized in that the cap (22) is axially adjustable relative to the holder (16).

17. Treatment instrument in accordance with claim 16, characterized in that the cap (22) can be screwed relative to the holder (16).

18. Electrosurgical treatment instrument in accordance with one of the preceding claims, characterized in that the cutting electrode (13) is removably arranged.

19. Treatment instrument in accordance with one of the preceding claims, characterized in that the front ring-like or sleeve-like electrode is the reference electrode (12).

20. Treatment instrument in accordance with one of the preceding claims, characterized in that the cutting electrode (13) is passed axially through the other two hollow electrodes (11, 12) and the insulation (20, 21) located therebetween.

21. Treatment instrument in accordance with one of the claims 18 to 20, characterized in that the cutting electrode (13) is attached via a plug connection (23, 25) to an axial feedline (25) fixedly arranged in the holder (16).

22. Treatment instrument in accordance with one of the preceding claims, characterized in that an insulating tube (33), through which the cutting electrodes (13) passes axially is inserted into the ring-like or sleeve-like front electrode (12).

23. Treatment instrument in accordance with one of the preceding claims, characterized in that two axially displaced hollow coagulation electrodes (11, 12), and within these a forwardly projecting cutting electrode (13), are arranged concentrically to one another.

24. Treatment instrument in accordance with one of the preceding claims, characterized in that at least one of the radio-frequency feed-line pairs (36, 39) is additionally energised with a low-frequency control current, which is kept away from the active electrode (11) by a high-pass filter (37), and is fed to a control switch arrangement (28, 28') provided at the holder (16), which is separated by a low-pass filter (40) from the radio-frequency and, by switching of the control current in the one or other direction, delivers a switching signal to a changeover device (35) connected to the radio-frequency generator (34), which applies the passing radio-frequency current to the coagulation electrode pair (11, 12) or to the cutting electrode pair (12, 13), depending on the direction of the control current.

25. Treatment instrument in accordance with claim 24, characterized in that the control current is an alternating current and the control switch arrangement has two switches (28, 28') connected in parallel and each lying in series with an oppositely switched rectifier (41, 42); and in that a switch arrangement is present in the changeover apparatus (35), which either energises the coagulation electrode pair (11, 12) or the cutting electrode pair (12, 13) with the radio-frequency current provided for it, depending on which of the two control switches (28, 28') is closed.

## Revendications

1. Instrument de traitement électrochirurgical comportant un manche (16 ; 16') susceptible d'être branché à un générateur haute fréquence (34), à l'extrémité proximale duquel sont prévues au moins deux électrodes de coagulation (11, 12 ; 11', 12') et une électrode de coupe (13) susceptibles d'être amenées en contact avec le tissu d'un patient, isolées les unes des autres et alimentées avec une tension à haute fréquence (H.F.) fournie par le générateur haute fréquence, caractérisé en ce que les deux électrodes (11, 12) sont réalisées en forme annulaire ou en forme de douille, agencées l'une dans l'autre et séparées l'une de l'autre par une couche de séparation isolante annulaire (20), les deux électrodes (11, 12) étant réalisées du moins en partie sur les surfaces latérales d'un corps en forme de tige ou de tenon (10) et la distance entre leurs surfaces extérieures étant si faible que lors de l'application des électrodes (11, 12) un courant de coagulation passe vers le tissu d'un patient, et en ce que l'électrode de coupe (13) est agencée dans l'espace intérieur des deux électrodes (11, 12) réalisées en forme de douille.

2. Instrument de traitement selon la revendication 1, caractérisé en ce que le corps (10) est sensiblement cylindrique.

3. Instrument de traitement selon la revendication 2, caractérisé en ce que le corps (10) est réalisé à l'extrémité proximale de manière à s'étendre en rétrécissement vers l'avant.

4. Instrument de traitement selon l'une quelconque des revendications précédentes, caractérisé en ce que les surfaces latérales du corps (10) sont réalisées comme des surfaces enveloppes lisses.

5. Instrument de traitement selon l'une quelconque des revendications précédentes, caractérisé en ce que les deux électrodes (11, 12) sont agencées l'une derrière l'autre.

6. Instrument de traitement selon la revendication 5, caractérisé en ce que l'électrode avant (12) est une bague métallique sphérique, parabolique ou conique et en ce que l'électrode arrière (11) est une douille métallique cylindrique.

7. Instrument de traitement selon la revendication 6, caractérisé en ce que l'électrode avant (12) et l'électrode arrière (11) sont concentriques l'une par rapport à l'autre.

8. Instrument de traitement selon l'une quelconque des revendications 5 à 7, caractérisé en ce qu'il est prévu une bague isolante (20) entre les électrodes (11, 12).

9. Instrument de traitement selon la revendication 8, caractérisé en ce que la bague isolante (20) est concentrique par rapport aux électrodes (11, 12).

10. Instrument de traitement selon l'une quelconque des revendications 5 à 9, caractérisé en ce que, à l'intérieur de l'électrode arrière (11) réalisée sous forme de douille, s'étend un prolongement tubulaire (21) de la bague isolante (20) qui est dirigé vers l'arrière, qui présente un diamètre réduit et dans lequel est agencé un tube métallique (17) qui s'étend depuis l'électrode avant (12) vers l'arrière.

11. Instrument de traitement selon la revendication 10, caractérisé en ce que le prolongement (21) dirigé vers l'arrière de la bague isolante (20) est agencé avec ajustement exact à l'intérieur de l'électrode arrière (11).

12. Instrument de traitement selon l'une ou l'autre des revendications 10 et 11, caractérisé en ce que le tube métallique (17) s'étendant depuis l'électrode avant (12) vers l'arrière est agencé avec ajustement exact dans le prolongement (21) de la bague isolante (20).

13. Instrument de traitement selon l'une quelconque des revendications précédentes, caractérisé en ce que l'électrode annulaire ou en forme de douille (11) est axialement réglable par rapport à l'électrode de coupe (13) agencée de façon axialement fixe sur le manche (16).

14. Instrument de traitement selon l'une quelconque des revendications précédentes, caractérisé en ce que les électrodes (11, 12) et les couches de séparation (20, 21) forment une unité structurelle conjointement avec les éléments de fixation, laquelle est agencée à l'extrémité proximale du manche (16).

15. Instrument de traitement selon la revendication 14, caractérisé en ce que l'unité structurelle est réalisée sous forme d'un capuchon (22).

16. Instrument de traitement selon l'une ou l'autre des revendications 9 et 10, caractérisé en ce que le capuchon (22) est axialement réglable par rapport au manche (16).

17. Instrument de traitement selon la revendication 16, caractérisé en ce que le capuchon (22) est vissable par rapport au manche (16).

18. Instrument de traitement selon l'une quelconque des revendications précédentes, caractérisé en ce que l'électrode de coupe (13) est agencée de façon détachable.

19. Instrument de traitement selon l'une quelconque des revendications précédentes, caractérisé en ce que l'électrode avant annulaire ou en forme de douille est l'électrode de référence (12).

20. Instrument de traitement selon l'une quelconque des revendications précédentes, caractérisé en ce que l'électrode de coupe (13) est menée axialement à travers les deux autres électrodes (11, 12) réalisées creuses et à travers l'isolation (20, 21) interposée.

21. Instrument de traitement selon l'une quelconque des revendications 18 à 20, caractérisé en ce que l'électrode de coupe (13) est agencée via une liaison enfichable (23, 25) sur une ligne d'amenée axiale (25) agencée solidairement dans le manche (16).

22. Instrument de traitement selon l'une quelconque des revendications précédentes, caractérisé en ce que dans l'électrode avant (12) annulaire ou en forme de douille est mis en place un tube isolant (33) à travers lequel s'étend axialement l'électrode de coupe (13).

23. Instrument de traitement selon l'une quelconque des revendications précédentes, caractérisé en ce qu'il est prévu concentriquement les unes aux autres, deux électrodes de coagulation creuses (11, 12) axialement décalées et à l'intérieur de celles-ci une électrode de coupe (13) dépassant à l'avant.

24. Instrument de traitement selon l'une quelconque des revendications précédentes, caractérisé en ce que l'une au moins des paires de lignes d'amenée à haute fréquence (36, 39) est attaquée en supplément par un courant de commande à basse fréquence qui est maintenu isolé de l'électrode active (11) par un passe-haut (37) et qui est amené à un agencement de commutation (28, 28') prévu sur le manche (16), ledit agencement étant isolé de la haute fréquence par un passe-bas (40) et fournissant un signal de commutation à un appareil inverseur (35) branché au générateur haute fréquence (34), par commutation du courant de commande dans l'une ou dans l'autre direction, ledit appareil appliquant le courant à haute fréquence approprié soit à la paire d'électrodes de coagulation (11, 12), soit à la paire d'électrodes de coupe (12, 13), en fonction de la direction du courant de commande.

25. Instrument de traitement selon la revendication 24, caractérisé en ce que le courant de commande est un courant alternatif et l'agencement de commutation comporte deux interrupteurs (28, 28') situés en série avec un redresseur respectif (41, 42) connecté en sens opposé et branchés parallèlement l'un à l'autre, et en ce qu'il existe dans l'appareil inverseur (35) un agencement de commutation qui attaque soit la paire d'électrodes de coagulation (11, 12), soit la paire d'électrodes de coupe (12, 13) par le courant à haute fréquence prévu à cet effet, en fonction de celui des deux interrupteurs de commande (28, 28') qui est fermé.
